# EUROPÄISCHE PATENTSCHRIFT

(11) **EP 0 721 511 B1**
(45) Veröffentlichungstag und Bekanntmachung des Hinweises auf die Patenterteilung: **22.11.2000**
(21) Anmeldenummer: 94927559.8
(22) Anmeldetag: 06.09.1994
(51) Int. Cl.: C12P 19/32, C07H 19/19, C07H 19/20, C12P 19/40, C12R 1/00

(54) **VERFAHREN ZUR HERSTELLUNG VON ARABINONUKLEOTIDEN**
METHOD OF PREPARING ARABINONUCLEOTIDES
PROCEDE DE PREPARATION D'ARABINONUCLEOTIDES

(30) Priorität: 28.09.1993 DE 4333727
(43) Veröffentlichungstag der Anmeldung: 17.07.1996
(73) Patentinhaber: SCHERING AKTIENGESELLSCHAFT, 13342 Berlin (DE)
(72) Erfinder: HUMMEL-MARQUARDT, Heidi, D-59368 Werne (DE); SCHMITZ, Thomas, D-10997 Berlin (DE); KENNECKE, Mario, D-14193 Berlin (DE); WEBER, Alfred, D-14169 Berlin (DE)
(86) Internationale Anmeldenummer: EP9402949
(87) Internationale Veröffentlichungsnummer: WO9509244

(56) Entgegenhaltungen:
- WO-A-92/09604
- DE-A- 1 517 836
- Patent Abstracts of Japan, Band 5, Nr 152(C-75); & JP,A,5682098 (AJINOMOTO K.K.), 1981-07-04
- Patent Abstracts of Japan, Band 7, Nr 219(C-188); & JP,A,58116698 (AJINOMOTO K.K.), 1983-07-11

## Beschreibung

Die Erfindung betrifft ein Verfahren zur Herstellung von Arabinonukleotiden der allgemeinen Formel I 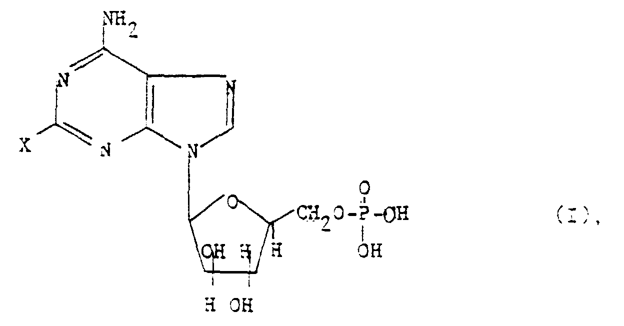 worin
X ein Wasserstoffatom oder ein Fluoratom darstellt, welches dadurch gekennzeichnet ist, daß man ein Arabinonukleosid der allgemeinen Formel II 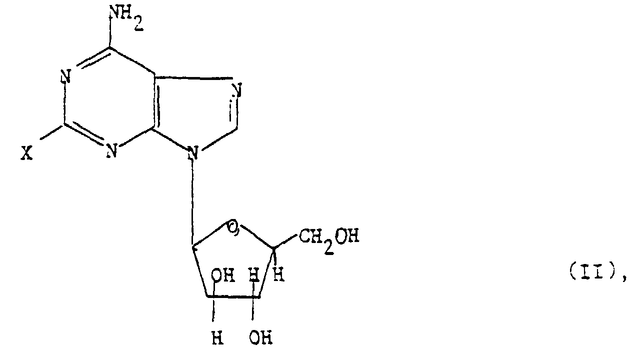 worin X die oben genannte Bedeutung besitzt, in Gegenwart eines Arylphosphats der allgemeinen Formel III 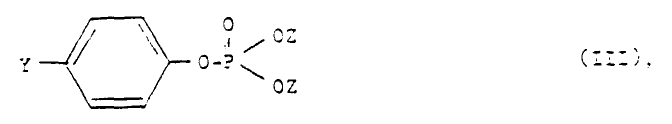 worin
Y ein Wasserstoffatom oder eine Nitrogruppe und
Z zwei Wasserstoffatome oder zwei Alkalimetallatome symbolisiert, mit einem zur Phosphorylierung von Nucleosiden befähigten Mikroorganismus fermentiert.

Die Arabinonucleotide der allgemeinen Formel I, das 9(5-0-Phosphono-β-D-arabinofuranosyl)-9-H-purin-6-amin (=Vidarabinphosphat) und das 2-Fluor-9-(5-0-phosphono-β-D-arabinofuranosyl)-9H-purin-6-amin (=Fludarabinphosphat) sind bekanntlich pharmakologisch wirksame Substanzen, die sich durch eine antivirale und cylostatische Wirksamkeit auszeichnen (EP-A 317,728 und WO 9209604).

Nach den bekannten Verfahren werden diese Verbindungen durch Phosphorylierung der entsprechenden Nucleoside hergestellt (Bull. of th. Chem. Soc. Japan 42, 1969, 3505-3508), New Journal of Chem. 11, 1987, 779-785 und WO 9209604). Bei diesem Verfahren erhält man stark verunreinigte Rohprodukte, deren Aufreinigung sehr aufwendig und verlustreich sind.

Aus der JP-A-58-116698 ist ferner ein mikrobiologisches Verfahren zur Herstellung von Purinnukleosid-monophosphat bekannt. Als Edukte werden in diesem Dokument weder ein Arabinonukleotid noch ein Arylphosphat beschrieben. In dem Verfahren wird kein Phosphatdonator verwendet, der auch in großem Überschuß verwendbar ist. Ein solcher Donator ist aber essentiell zum Abbruch der mikrobiologischen Reaktion und zum Vermeiden von störenden Isomeren-Gemischen.

Das erfindungsgemäße Verfahren ermöglicht es, diese Substanzen in relativ einfacher Weise in wesentlich reinerer Form zu synthetisieren, als dies mittels der vorbekannten Verfahren möglich ist.

Dies ist für den Fachmann überraschend. Aus den Untersuchungen von Koji Misugi et al. (Agr.Biol.Chem. 28, 1964, 586-600) ist zwar schon lange vorbekannt, daß man das Nucleosid Inosin mikrobiologisch phosphorylieren kann, man mußte aber erwarten, daß man bei der Phosphorylierung der Nucleoside der allgemeinen Formel II wesentlich ungünstigere Ergebnisse erzielen würde. Dies insbesondere aus zwei Gründen: Aus den Arbeiten v on Koji Mitsugi et al. ist bekannt, daß man bei der Phosphorylierung von Inosin oft Gemische isomerer Nucleotide erhält. Es war demzufolge zu erwarten, daß man bei der Phosphorylierung der Nucleoside der allgemeinen Formel II im gleichen - wenn nicht sogar im verstärkten Maße Isomerengemische erhalten würde.

Es ist bekannt, daß Adenosin im Körperstoffwechsel unter Desamierung und Oxidation abgebaut wird (Römpps Chemie-Lexikon, 8te Auflage, Franckh'sche Verlagshandlung, Stuttgart (DE) 65) und es war demnach zu befürchten, daß bei der mikrobiologischen Umsetzung der Adenin-Derivate der allgemeinen Formel II zumindest partiell ebenfalls ein Abbau der Verbindungen eintreten würde.

Wie eigene Versuche, die mit dem auch von Koji Mitsugi et al. erwähnten Mikroorganismus Pseudomonas trifolii (IAM 1309 nach einer Untersuchung des DSM-Identifikationsdienstes ist seine ist er heute als Pantoeaagglomerans eingeordnet) treten die geschilderten befürchteten Nachteile bei der Phosphorylierung der Nucleoside der allgemeinen Formel II nicht auf, sondern die Reaktion scheint sogar noch günstiger zu verlaufen als diejenige des Inosins.

Mit hoher Wahrscheinlichkeit kann das erfindungsgemäße Verfahren nicht nur mit dem getesteten Mikroorganismus Pseudomonas trifolii (IAM 1309), sondern auch mit anderen Mikroorganismen, die von Koji Mitsugi et al. als zur Phosphorylierung von Nucleosiden als geeignet beschrieben sind, durchgeführt werden. Es sind dies beispielsweise die Mikroorganismen:
- Pseudomonas trifolii: IAM 1543 und IAM 1555
- Pseudomonas perlurdia: IAM 1589, IAM 1600, IAM 1610 und IAM 1627,
- Pseudomonas melanogenum: F-11,
- Alcaligenes visco lactis: ATCC 9039 und IFM AN-14,
- Achromobacter superficialis: IAM 1433
- Flavobacterium lactis: IFM F101
- Flavobacterium fuscum: IAM 1181
- Flavobacterium flavescens: IFO 3085
- Flavobacterium breve: IFM S-15
- Serracina marcescens: IAM 1022, IAM 1065, IAM 1067, IAM 1104, IAM 1135, IAM 1161, IAM 1205, IAM 1223, IAM 1703
und weitere Mikroorganismen, die in dieser Publikation aufgeführt sind.

Das erfindungsgemäße Verfahren muß, um eine ausreichende Phosphorylierung der nur sehr aufwendig herstellbaren Arabinonucleoside der allgemeinen Formel II zu erzielen, in Gegenwart eines großen Überschusses an einem Phosphatdonator durchgeführt werden. Geeignete Phosphatdonatoren sind unter anderem Arylphosphate, wie das Phenylphosphat oder das p-Nitrophenylphosphat. Üblicherweise verwendet man pro mol umzusetzendem Nucleosid 2-5 mol Phosphatdonator.

Da die in Bakterien vorkommenden Proteasen meist alkalische Proteasen sind, die Zinkproteine darstellen und meist Magnesiumionen zur Entfaltung ihrer Wirksamkeit benötigen, ist es zweckmäßig, die Umsetzung in Gegenwart von 0,2 bis 4,0 % eines wasserlöslichen Zinksalzes, wie zum Beispiel Zinksulfat-Dihydrat oder gegebenenfalls auch in Gegenwart von 0,01 bis 0,3 % eines wasserlöslichen Magnesiumsalzes, wie Magnesiumsulfat-Heptahydrat durchzuführen.

Abgesehen von den genannten Bedingungen kann das erfindungsgemäße Verfahren unter den gleichen Bedingungen durchgeführt werden, die man üblicherweise bei der Fermentation von Substraten mit Bakterienkulturen anwendet. Die Bakterienkultur wird in einem geeigneten Medium angezüchtet, das Substrat und die Hilfsstoffe zugesetzt und die Kultur unter Rühren und Belüften fermentiert,bis eine maximale Substratumwandlung erreicht wird.

Wendet man dieses Verfahen an, so erhält man in der Regel Fermentationsbrühen, bei denen das Verfahrensprodukt nur schwer vom Fermentationsmedium abtrennbar ist.

Deshalb erscheint es in der Regel zweckmäßiger, die Reaktion unter den Bedingungen des resting-cell-Verfahrens durchzuführen. Zu diesem Zwecke werden die Bakterienkulturen in einem üblichen Medium angezüchtet, die Bakterien durch Zentrifugieren abgetrennt, gewaschen, eventuell gefriergetrocknet - um sie lagerfähig zu machen - in einer isotonischen Pufferlösung resuspendiert, mit Substrat und Hilfsstoffen versetzt und bei 20 bis 40°C fermentiert, bis eine maximale Substratumwandlung erreicht ist. Unter diesen Gegebenheiten bereitet die Aufbereitung des Ansatzes keine Schwierigkeiten, das Zellmaterial wird abzentrifugiert, der Überstand eingeengt und das ausgefallene Verfahrensprodukt, welches durch leicht abtrennbares Ausgangsmaterial verunreinigt sein kann, abfiltriert.

Die nachfolgenden Ausführungsbeispiele dienen zur Erläuterung des erfindungsgemäßen Verfahrens:

### Beispiel 1

a) Eine Petrischale mit einem Medium enthaltend
   1 % Pepton
   0,2 % Hefeextrakt
   0,1 % Magnesiumsulfat-Heptahydrat und
   1,5 % Agar
      - eingestellt auf pH 7.0 -
   wird mit einer Trockenkultur von Pseudomonas trifolii IAM 1309 beimpft und 16 Stunden bei 30° lang inkubiert.
b) Ein 2 l Erlenmeyerkolben mit 1 l Medium enthaltend
   1 % Pepton
   0,2 % Hefeextrakt und
   0,1 % Magnesiumsulfat-Heptahydrat
      - eingestellt auf pH 7-
   wird mittels einer Öse mit der gemäß a) hergestellten Vorkultur beimpft und 16 Stun den lang bei 30°C mit 180 Umdrehungen pro Minute inkubiert. Dann werden die Zellen 15 Minuten lang mit 6000 Umdrehungen pro Minute bei 10°C abzentrifugiert, mit 200 ml einer 0,02%igen wässrigen Kaliumchloridlösung gewaschen, in 20 ml einer 0,02%igen wässrigen Kaliumchloridlösung suspendiert, bei -20°C eingefroren und 20 Stunden lang gefriergetrocknet. Zum Gebrauch werden die gefriergetrockneten Zellen bei Raumtemperatur gelagert.
c) In Schraubflaschen werden in 0,5 M Natriumacetatpuffer von pH 4,5 pro l
   2,0 g 2-Fluor-9-(β-D-arabinofuranosyl)-9H-purin-6-amin,
   0,12 g Zinksulfat-Dihydrat,
   1,0 g gemäß Beispiel 1b hergestellte gefriergetrocknete Pseudomonas trifolii IAM 1309 Kultur und
   8,0 g Dinatrium-p-nitrophenylphosphat
   eingetragen und die Reaktionsmischen 40 Stunden lang bei 40°C mit 60 Umdrehungen pro Minute geschüttelt.
Dann zentrifugiert man die Zellen mit 8000 Umdrehungen pro Minute ab, engt den Überstand im Rotationsverdampfer bei maximal 50°C auf 1/20 des ursprünglichen Volumens ein, filtriert das abgeschiedene Rohprodukt ab, wäscht es mit Wasser und trocknet es 24 Stunden lang bei 100°C und 10000Pa.
Laut HPLC Analyse des erhaltenen Rohprodukts sind bei diesem Versuch ca. 50% 2-Fluor-9-(5-0-phosphono-β-D-arabinofuranosyl)-9H-purin-6-amin gebildet worden.

### Beispiel 2

Unter den Bedingungen des Beispiels 1c, aber unter Zusatz von 16g/l Dinatrium-p-nitrophenylphosphat anstelle von 8g/l,werden 2.0 g/l 2-Fluor-9(β-D-arabinofuranosyl)-9H-purin-6-amin 40 Stunden lang bei 40°C mit 60 Umdrehungen pro Minute geschüttelt. Die Aufarbeitung der Reaktionsmischung erfolgt wie in Beispiel 1c beschrieben und man erhält ca. 60% 2-Fluor-9-(5-0-phosphono-β-D-arabinofuranosyl)-9H-purin-6-amin.

### Beispiel 3

Unter den Bedingungen des Beispiels 1c aber unter Zusatz von 40g/l Dinatrium-p-nitrophenylphosphat anstelle von 8g/l,werden 2,0 g/l 2-Fluor-9(β-D-arabinofuranosyl)-9H-purin-6-amin 100 Stunden lang bei 40°C mit 60 Umdrehungen pro Minute geschüttelt. Die Aufarbeitung der Reaktionsmischung erfolgt wie in Beispiel 1c beschrieben und man erhält ca. 85% 2-Fluor-9-(5-0-phosphono-β-D-arabinofuranosyl)-9-H-purin-6-amin.

### Beispiel 4

Unter den Bedingungen des Beispiels 1c aber unter Zusatz von 20g/l Dinatriumphenylphosphat anstelle von 8g/l Dinatrium-p-nitrophenylphosphat,werden 2,0g/l 2-Fluor-9(β-D-arabinofuranosyl)-9H-purin-6-amin 100 Stunden lang bei 40°C mit 60 Umdrehungen pro Minute geschüttelt. Die Aufarbeitung der Reaktionsmischung erfolgt wie in Beispiel 1c beschrieben und man erhält ca. 50% 2-Fluor-9-(5-0-phosphono-β-D-arabinofuranosyl)-9H-purin-6-amin.

### Beispiel 5

Unter den Bedingungen des Beispiels 4 aber unter Zusatz von 30g/l Dinatriumphenylphosphat anstelle von 20g/l, werden 2,0g/l 2-Fluor-9-(β-D-arabinofuranosyl)-9H-purin-6-amin umgesetzt, aufbereitet und man erhält 60% 2-Fluor-9-(5-0-phosphono-β-D-arabinofuranosal)-9-H-purin-6-amin.

### Beispiel 6

Unter den Bedingungen des Beispiels 4 aber unter Zusatz von 40g/l Dinatriumphenylphosphat anstelle von 20g/l,werden 2,0g/l 2-Fluor-9-(β-D-arabinofurynosyl)-9H-purin-6-amin umgesetzt, aufbereitet und man erhält 70% 2-Fluor-9-(5-0-phosphono-β-D-arabinofuranosyl)-9H-purin-6-amin.

## Patentansprüche

1. Verfahren zur Herstellung von Arabinonukleotiden der allgemeinen Formel I 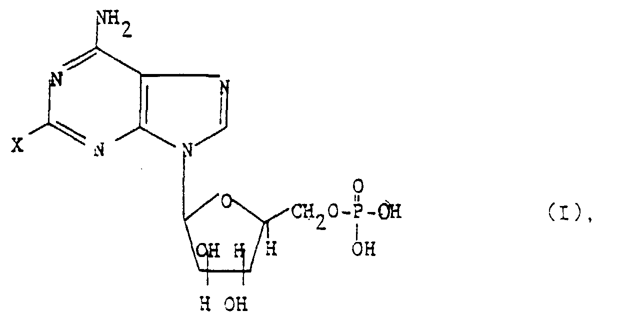 worin
X ein Wasserstoffatom oder ein Fluoratom darstellt, welches dadurch gekennzeichnet ist, daß man ein Arabinonukleosid der allgemeinen Formel II 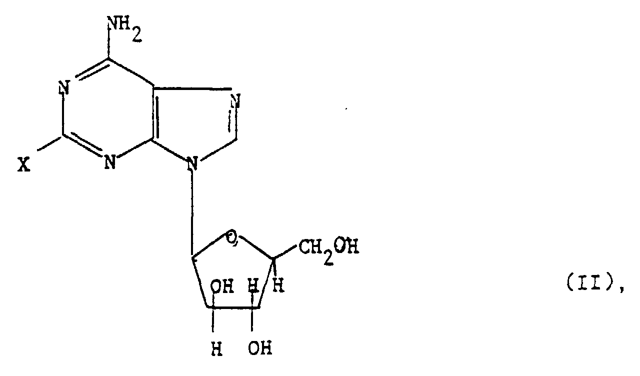 worin X die oben genannte Bedeutung besitzt, in Gegenwart eines Arylphosphats der allgemeinen Formel III 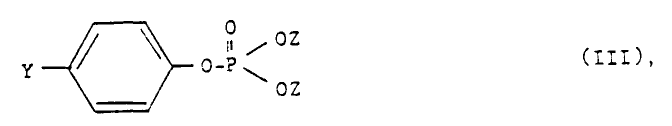 worin
Y ein Wasserstoffatom oder eine Nitrogruppe und
Z zwei Wasserstoffatome oder zwei Alkalimetallatome symbolisiert, mit einem zur Phosphorylierung von Nucleosiden befähigten Mikroorganismus fermentiert.

2. Verfahren zur Herstellung von Arabinonukleotiden der allgemeinen Formel I gemäß Patentanspruch 1, dadurch gekennzeichnet, daß man die Fermentation in Gegenwart eines wasserlöslichen Zink(II)-salzes durchführt.

3. Verfahren zur Herstellung von Arabinonucleotiden der allgemeinen Formel I gemäß Patentanspruch 1 und 2, dadurch gekennzeichnet, daß man die Fermentation unter den Bedingungen des resting cell-Verfahrens durchführt.

4. Verfahren zur Herstellung von Arabinonukleotiden der allgemeinen Formel I gemäß Anspruch 1 bis 3, dadurch gekennzeichnet, daß man zur Fermentation einen Mikroorganismus der Spezies Pseudomonas trifolii verwendet.

5. Verfahren zur Herstellung von Arabinonukleotiden der allgemeinen Formel I gemäß Patentanspruch 1 bis 4, dadurch gekennzeichnet, daß man zur Fermentation einen Mikroorganismus der Spezies Pseudomonas trifolii IAM 1309 verwendet.

## Claims

1. Process for the preparation of arabinonucleotides 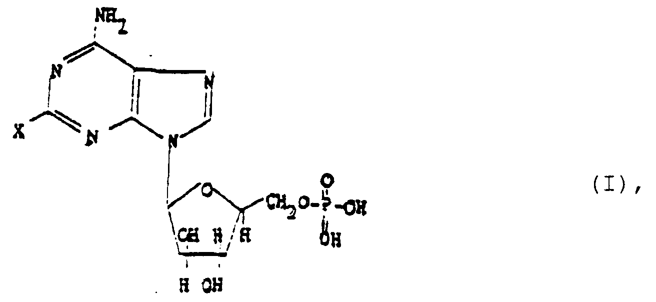 of the general formula I in which
X is a hydrogen atom or a fluorine atom, which is characterized in that an arabinonucleoside of the general formula 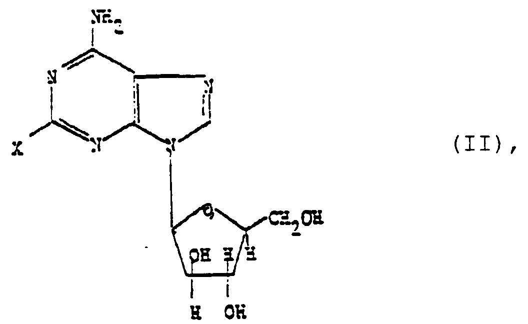 II in which X has the abovementioned meaning is fermented in the presence of an aryl phosphate of the general formula III 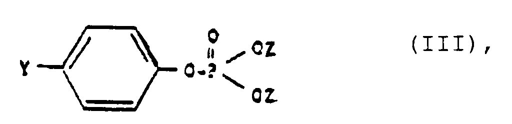 in which
Y symbolizes a hydrogen atom or a nitro group and
Z symbolizes two hydrogen atoms or two alkali metal atoms,
using a microorganism capable of the phosphorylation of nucleosides.

2. Process for the preparation of arabinonucleotides of the general formula I according to Patent Claim 1, characterized in that the fermentation is carried out in the presence of a water-soluble zinc(II) salt.

3. Process for the preparation of arabinonucleotides of the general formula I according to Patent Claim 1 and 2, characterized in that the fermentation is carried out under the conditions of the resting-cell process.

4. Process for the preparation of arabinonucleotides of the general formula I according to Claim 1 to 3, characterized in that a microorganism of the species Pseudomonas trifolii is used for the fermentation.

5. Process for the preparation of arabinonucleotides of the general formula I according to Patent Claim 1 to 4, characterized in that a microorganism of the species Pseudomonas trifolii IAM 1309 is used for the fermentation.

## Revendications

1. Procédé de préparation d'arabinonucléotides de formule générale I 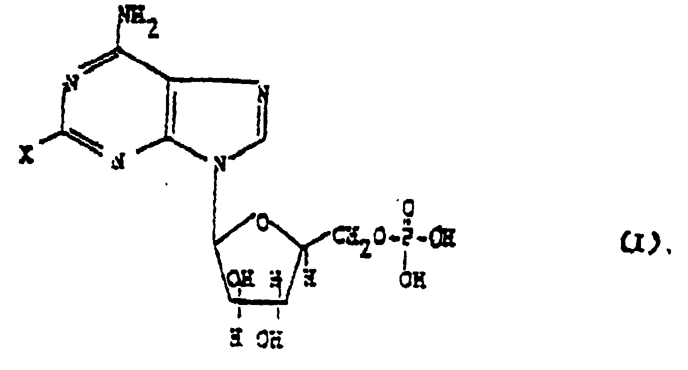 dans laquelle
X représente un atome d'hydrogène ou un atome de fluor,
qui est caractérisé en ce qu'on fermente avec un micro-organisme rendu capable de phosphoryler des nucléosides un arabinonucléoside de formule générale (II) 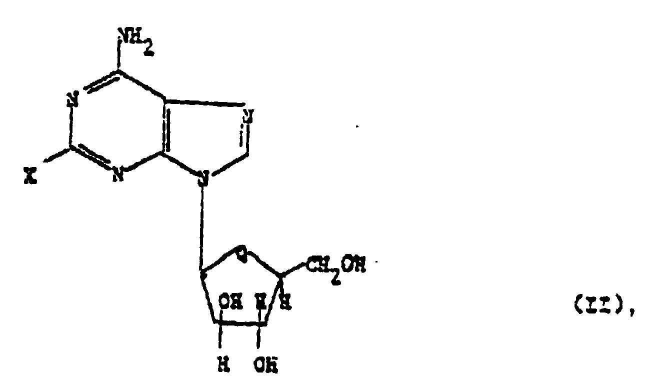 dans laquelle X possède la signification susmentionnée, en présence d'un arylphosphate de formule générale (III) 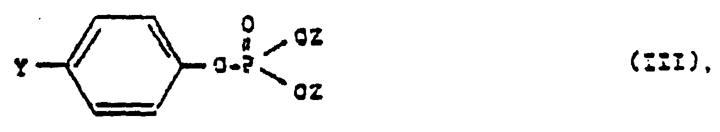 dans laquelle
Y symbolise un atome d'hydrogène ou un groupement nitro et
Z symbolise deux atomes d'hydrogène ou deux atomes de métal alcalin.

2. Procédé de préparation d'arabinonucléotides de formule générale I suivant la revendication 1, caractérisé en ce qu'on effectue la fermentation en présence d'un sel de zinc(II) soluble dans l'eau.

3. Procédé de préparation d'arabinonucléotides de formule générale I suivant les revendications 1 et 2, caractérisé en ce qu'on effectue la fermentation dans les conditions du procédé en cellules restantes.

4. Procédé de préparation d'arabinonucléotides de formule générale I suivant les revendications 1 à 3, caractérisé en ce qu'on utilise pour la fermentation un micro-organisme de l'espèce Pseudomonas trifolii.

5. Procédé de préparation d'arabinonucléotides de formule générale I suivant les revendications 1 à 4, caractérisé en ce qu'on utilise pour la fermentation un micro-organisme de l'espèce Pseudomonas trifolii IAM 1309.
